# EUROPEAN PATENT SPECIFICATION

(11) **EP 4 099 863 B1**
(45) Date of publication and mention of the grant of the patent: **06.12.2023**
(21) Application number: 21707368.3
(22) Date of filing: 29.01.2021
(51) Int. Cl.: A43B 17/00, A43B 13/38, A61F 5/14, A43B 7/14

(54) **PLANTAR ORTHOSIS**
SOHLENORTHESE
ORTHÈSE PLANTAIRE

(30) Priority: 03.02.2020 IT 202000002008
(43) Date of publication of application: 14.12.2022
(73) Proprietor: Corona, Roberto, 67051 Avezzano (AQ) (IT)
(72) Inventor: Corona, Roberto, 67051 Avezzano (AQ) (IT)
(74) Representative: Leone, Mario
(86) International application number: PCT/IB2021/050715
(87) International publication number: WO 2021/156717

(56) References cited:
- US-A1- 2007 033 834
- US-A1- 2009 320 330
- US-A1- 2011 247 235
- US-A1- 2016 313 174
- US-A1- 2019 200 699

## Description

### BACKGROUND OF THE INVENTION

### 1. Field of the invention

The present invention relates to a plantar orthosis, i.e. a medical device which is used outside a user's body with the purpose of modifying the structural or functional features of the neuro-musculo-skeletal apparatus, according to the general definition introduced by the International Organization of Standards.

Generally, under orthosis, then devices are meant which are used as apparatuses applicable to the body with corrective functions, but not as replacement of missing parts, differently from protheses. Generally, an orthosis has the function of achieving a relative immobilization of an articulation or limb suffering, for example, from trauma, pathologies, sprains of ligaments or which underwent a surgical operation and then it is an external instrument used in the treatment of some dysfunctions to help the patient in the functionalities of a limb thereof. Another use of the orthoses is the one concomitant with rehabilitation or functional re-education. The orthosis, at last, can be used to reduce the load on an articulation and/or limb and to decrease the related pain.

Plantar orthoses, or simply insoles, then are called the orthoses which are used at the foot sole; they are podalic supports which are inserted inside footwear and aimed at modifying the foot bone-articular ratios to obtain a morphous-structural re-equilibrium of the limb mainly depending upon the binding reaction provided by the ground during the resting and propulsion phase.

The plantar orthosis then is suitable to be used both as plantar inner sole and as device integrated in the sole of footwear, which in turn could be footwear intended to special uses, such as footwear planned for the exercise of particular sporting activities, or footwear devised for long marches and so on.

### 2. Description of the state of art

The insoles substantially are therapeutic orthoses since, generally, they are used under all circumstances wherein the foot complaints of a suffering. In particular they are used in case of full-blown pains, localized at the plantar level, with the pain treatment by means of a discharge of the hyper-pressure areas, or in case of systemic pathologies such as, for example, advanced-stage arthrosis, deforming arthritis, diabetes, serious circulatory insufficiency, or in the need for correcting pathological postures, such as pronations, supinations, shortening, dysmetria, and so on.

The plantar orthoses can be classified, based upon their functional features, in corrective or functional insoles and antalgic insoles or insoles with increased comfort.

The insoles used for corrective purposes set the goal of correcting problems caused by a wrong plantar rest, mainly in the age of development, that is until the muscle-tendon-skeletal apparatus is capable of responding to external stresses.

Some typical problems, which are faced with this type of orthosis, are rear foot pronation, genu valgum, flatfoot or forefoot varus. In order to support such corrective purposes, the functional plantar orthoses act by limiting or preventing determined podalic motions.

The antalgic insoles, on the contrary, circumscribe their action at the level of the plantar rest and they are devised with the purpose of reducing, limiting or even removing the pain caused after an ongoing problem through the increase in the resting surfaces.

Usually, the antalgic insoles are made of soft materials, to dampen or discharge the podalic rest in the areas having more pressure load or in a determined painful point, but such dampening, apart from dissipating plantar pressure, even involves a certain dissipation of the forces required to an effective pace which usually would be transmitted on the kinetic chain and the total absence of control of the foot mechanics.

Here, even the proprioceptive or sensorimotor insoles can be mentioned which, depending upon suitable projecting elements with respect to the plantar resting surface, intend to stress the proprioceptive sensitivity of the sensorial elements placed on the foot sole (proprioceptors) capable of triggering induced reflexes and thus modifying the postural response.

Generally, for implementing in a customized way the currently used insoles, an accurate anamnesis of the user is required and it is necessary to evaluate carefully the subject's postural and walking features, to detect the podalic footprint, the pressure loads and the areas subjected to higher load apart from detecting the specific foot cast.

The materials used for the plantar orthoses generally can be the most disparate ones, they can be grouped in the following categories: stiff; half-stiff; and soft. Among these types of materials we can mention, for example, carbon, the polymers, the so-called high-temperature thermoplastic materials, such as polyethylene, polypropylene, copolymers - the main advantage thereof is to keep a memory capable of making them to go back in the starting configuration after a deformation, and which are further very long-lasting and resistant - the low-temperature thermoplastic materials such as plastazote, which are used as protective layers to the orthosis-user interface, thermoformable composite resins, lattice and polyphenolic foams.

The methods therewith a plantar orthosis is manufactured currently are mainly two: production on cast and production with removal of material implemented with CAD-CAM method.

For the insoles on cast, the foot cast is acquired, obtained under conditions of static load or total discharge, and on such cast the insole is modelled, constructed through the overlapping of one or more homogeneous and undifferentiated flat layers of gum-like materials, adapted, through vacuum thermoforming, to the cast shape of the user's foot.

As far as the methods for removing material with CAD-CAM method are concerned, after having obtained a 2D and 3D scanning of the foot one proceeds with correcting the images by software. Subsequently, the footprint is sent to numerical control cutters which implement the insole by processing plates of gum-like materials having preferential density and consistency by obtaining the insole by automatized extrusion. The obtained orthoses are quite simple, often formed by only one layer of material, shaped with differentiated thicknesses but in this production method one succeeds in simplifying and shortening very much the processing phase time.

In conclusion, a conventional orthosis or of standard type will have different features and composition to adapt to the specific patient and will perform diversified supports and discharges between forefoot and backfoot based upon the objective which one wants to reach from the point of view of remodelling of the podalic pressure loads.

All known insoles can be considered mainly neutral and static, with respect to the posture dynamic character, that is they act on stresses which are substantially normalized, that is which can be considered exclusively vertical and have constant intensity in time.

Indeed, due to their constructive features and their intrinsic properties, these insoles are not capable of producing any dynamic action, in the sense of providing different and differentiated actions of response to the several dynamic stresses existing on the plantar arch during dynamic motions.

The insoles of traditional type simply respond to the need for decreasing the painful forms appearing on the foot sole, by providing the widest resting surface and by dividing the body weight as homogeneously as possible, or by inducing posture corrections by forcibly imposing some positions considered corrective through the width limitation of the podalic motion.

Such forced corrections, by altering the free carrying out of natural podalic motions during deambulation - according to many physicians and scholars - would induce anomalies in deambulation, thus causing inevitably the occurrence of additional alterations along the whole kinetic chain formed by foot, leg, pelvis, back, for the compensation natural phenomenon.

The technical problem underlying the present invention is to provide a plantar orthosis allowing to correct the altered and not correct postural states without limiting or preventing the free carrying out of the podalic helix dynamics, but by encouraging and supporting the correct carrying out thereof and consequently by inducing the restoring of the correct muscle and motor activations in the whole kinematic chain by producing naturally the postural rebalancing of the individual. The US patent application Nr. 2011/0247235 A1 describes a plantar orthosis comprising, in its thickness, a stabilization element the purpose thereof is to make stiffer the sole in transverse direction.

To this purpose, it can include a layer made of stiff material, or elements extending longitudinally like a spine, or still a matrix of annular structures, or with other shape, extending on the inner sole plane.

The presence of this stabilization element then contributes in varying the stiffness and flexibility of the plantar orthosis according to different directions.

The US patent application Nr. 2019/0200699 A1 describes another plantar orthosis comprising a structure constituted by one or more reinforcing yarns sewn in the thickness of the inner sole, to provide it a greater resistance, a greater longitudinal stiffness and a support for an upper layer.

### SUMMARY OF THE INVENTION

As just mentioned, an ideal configuration for a plantar orthopaedic device, which could go beyond the capability of the current conventional orthoses, should allow the suitable modifications in the foot trim and in its internal bearing structures, by optimizing them, thanks to a dynamic elastic approach, through their guided management, aimed at making the foot to assume the right resting configurations depending upon the different motions required to the whole kinematic chain. Such considerations are amplified whenever one enters areas of extreme physical use, such as for example in sport or military field.

The present invention then relates to plantar orthoses according to independent claim 1. Additional embodiments are defined in the dependent claims.

More in particular, the invention relates to plantar orthopaedic devices customized as to their specific use and the user's needs, which allow to control the stresses existing on the whole kinematic chain through the suitable management of the forces and moments acting on the plantar surface through guided and controlled deformations - along defined and determined directrices - of the orthosis itself and the management of the elastic energies at play.

Such result implements through the implementation of targeted and specific thickening of the orthosis cross sections, obtained by overlapping not homogeneously layers of materials having different densities, different thicknesses and different mechanical features, with the purpose of generating, during the evolution of the podalic motion, inhomogeneous deformations of the orthosis surface which, consequently, generate complex motions of the orthosis itself and guide the limb to assume the right evolution according to the natural dynamics of the podalic motion.

According to such technique it is possible to define even privileged directrices for transferring the elastic energy components which have developed during the motion inside the orthosis so as to collect them from some areas and to release them in different, suitably detected areas, with the purpose of helping and potentiating therewith the right podalic motions.

The application of such approach has been developed in the field of the sporting activities and military actions, constituting two among the most extreme use scenarios, without these fields resulting in any case to be limiting in relation to the use of such approach in all other imaginable fields.

In the design scenarios, the implemented objectives are the drastic reduction in the risk of occurrences of muscle, tendon, joint injuries and injuries of the ligaments due to overload and stress apart from the long-term damages due to wear.

Such result is obtained by generating, by reason of the described podalic motion imposed by the orthosis and the energy management, the correct motor activations along the whole kinematic chain.

The technical problem underlying the present invention is then solved by a plantar orthosis as specified above, characterized as detailed in the enclosed claim 1, whereas additional accessory details are defined in the enclosed depending claims.

In these plantar orthoses a concept behind production is applied according thereto one aims at eliminating the causes of injury and/or damage, by working first of all with a preventive approach therefore not only and not so much the problems linked to the foot are considered, but evaluations are extended to the whole kinematic chain thereof the foot is a component.

The interest, then, is addressed not only to the plantar dynamics, but even and above all to the way in which such dynamics are correlated to the musculoskeletal dynamics of the whole lower limb.

This new plantar orthosis is based upon a logic which modifies the classical approach of the traditional plantar orthoses which are based upon the static consideration of the vertical (pressure) components only of the plantar rest. The stresses and relative energies, acting on the plantar rest, are now considered as complex dynamic loads having not only vertical components with respect to the ground, but even cutting components and rotary components and which do not appear statically with constant intensity over time, but they show a recurrent variation in intensity of the components and a continuous shifting of the application points and of the developing directions.

Moreover, such plantar loads are related to those acting on other proximal elements of the whole lower limb and they condition all motor activities along the kinematic chains by determining and managing the forces at play thereof.

Controlling and managing the plantar stresses then means the fact of succeeding in managing stresses on the entire rest of kinematic chains, and then controlling the most harmful and most dangerous components which could determine the occurrence of injuries and/or damages on the whole musculoskeletric system of the user.

The plantar orthosis according to the invention is capable of widening the management of its own elastic response, even thanks to a specific customized planning of its structural components.

The characteristic structural components of the orthosis are then devised to translate the dynamic stresses on the plantar surface to generate and manage differentiated deformations of the plantar orthosis under load so as to support and guide the correct podalic motion and to transfer mechanical energy between the different points of the resting foot along defined predetermined directrices.

In particular, the structural components of the orthosis, apart from supporting the complex motion of the foot in the resting phase, by reason of their mechanical features of differentiated deformation, accumulate energy in the points of maximum negative intensity, under form of elastic potential energy, and transfer it in other areas of the plantar rest, by releasing them in the areas wherein such elastic energy favours the activation of motor components favourable to motion.

By managing suitably the stresses on the plantar rest it is possible, by means of these new plantar orthoses, to manage and control the motor activation of the body segments of the upper tract and to control the stresses along the whole kinematic chain, by performing a substantial neutralization of the situations more at risk of injury.

Therefore, in the plantar orthosis according to the invention, the use of structural elements having different geometries and peculiar mechanical features is provided, which overlapped and assembled therebetween are capable of determining heterogeneous and non-linear mechanical features in the different cross sections, in turn capable of generating non-linear deformations of the whole orthosis.

Such feature could later be obtained by using special materials which in plates having uniform thickness are capable of having differentiated mechanical features among the different areas (for example through the 3D printing technique and more).

Among such structural elements there is a dynamic element inserted among the other layers composing the orthosis, in particular between a base reinforcement layer and a lower layer.

Such dynamic element is implemented by a continuous belt-like strip having a predetermined thickness and width and which is arranged near the external edge of the heel portion, and which then extends with one or more belt-like branches as far as respective front resting portions of the plantar orthosis, in particular as far as arriving at the external midfoot and near the hallux of said plantar arch.

This continuous dynamic element is made of a material which is more deformable than the material of the layers enclosing it.

Moreover, the dynamic element, that is said continuous belt-like strip is capable of deforming elastically and of varying its own sizes according to different directions, due to the effect of the non-linear action of the orthosis deformation due to the helical winding/unwinding motion of prono-supination of the foot, by generating inside thereof a pressure elastic wave along its length and going back later to its own natural shape.

In this way, the dynamic element cooperates with the other structural elements of orthosis in transforming and accumulating the energy due to the impact with the ground under form of elastic potential energy and it is capable of transferring, addressing and releasing such energy from the heel portion to the metatarsus portion and vice versa according to the preferential privileged directrices.

### BRIEF DESCRIPTION OF THE DRAWINGS

The present invention will be described hereinafter according to a preferred embodiment thereof, provided by way of not limiting example with reference to the enclosed drawings wherein:

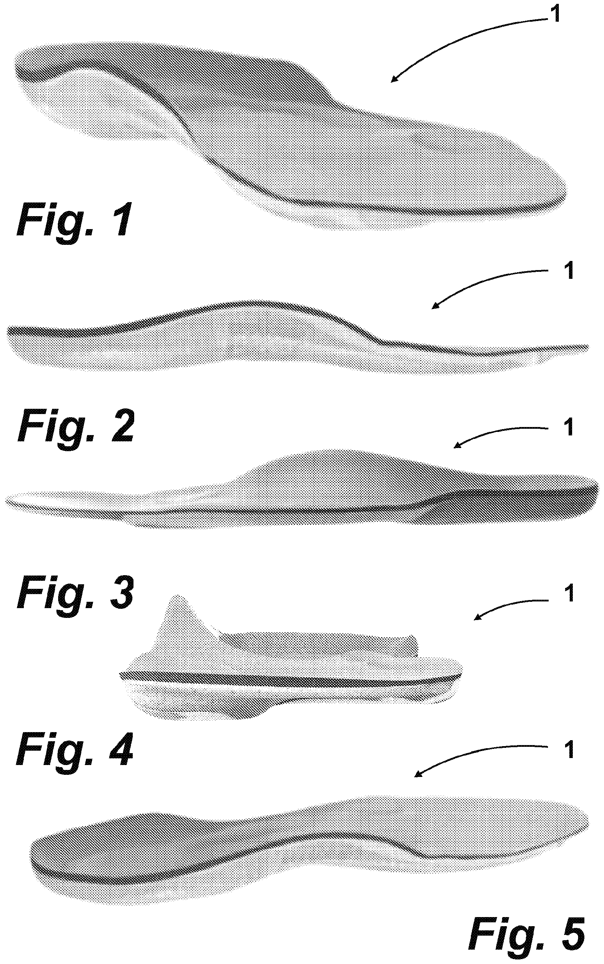

* figure 1 shows a top front perspective view of a plantar orthosis according to the present invention;
* figure 2 shows a right side view of the plantar orthosis of figure 1;
* figure 3 shows a left side view of the plantar orthosis of figure 1;
* figure 4 shows a front view of the plantar orthosis of figure 1;
* figure 5 shows a rear perspective view from top of the plantar orthosis of figure 1;
* figure 6 shows an exploded perspective view of the plantar orthosis of figure 1, which illustrates the components thereof;
* figure 7 shows a top schematic view of a first embodiment example of plantar orthosis according to the invention, illustrating in transparency the different components;
* figure 8 shows a detailed view of one of said components of the plantar orthosis of figure 7;
* figure 9 shows a top schematic view of a second embodiment example di plantar orthosis according to the invention, illustrating in transparency the different components;
* figure 10 shows a detailed view of one of said components of the plantar orthosis of figure 9;
* figures 11A, 11B and 11C illustrate the various running phases of a user of the plantar orthosis of the preceding figures;
* figures 12A, 12B, 12C, 12D and 12E illustrate schematically the course of some dynamic stresses in an elastic component of the plantar orthosis of the preceding figures in the rectilinear accelerated running motion;
* figures 13A, 13B, 13C, 13D, 13E, 13F and 13G illustrate schematically the course of the dynamic stresses in an elastic component of the plantar orthosis of the preceding figures on limb inside the motion of direction change.
* figures 14A, 14B, 14C, 14D, and 14E illustrate schematically the course of the dynamic stresses in an elastic component of the plantar orthosis of the preceding figures on limb outside the motion of direction change.
* figure 15 illustrates schematically an example for reinforcing the arch of the plantar orthosis of the preceding figures.
* figures 16A, 16B, 16C, 16D, 16E, 16F and 16G illustrate the typical deformation types of the plantar orthosis in the several sub-phases of the Resting (Contact, Intermediate, Transition and Push Intermediate) motion according to the modes inherent and characteristic of the orthosis itself.

With reference to the figures, a plantar orthosis is designated as a whole with 1, formed by a plurality of overlapped layers which will be described hereinafter. These layers constitute half-manufactured components developed dimensionally in a computerized way through a procedure for cutting a substrate with numerical control plotter. Moreover, such components can be obtained even through digital printing with 3D printers.

### DESCRIPTION OF SOME EMBODIMENT EXAMPLES OF THE INVENTION

The orthosis one comprises a base reinforcement layer, which hereinafter can be simply designated as base or frame of the orthosis, designated with 5, extending dimensionally over almost the whole extension of the podalic rest, or at least from the heel to the metatarsal area, and then receives on its surface the backfoot and the midfoot regions. The base reinforcement layer 5, which is in an intermediate position with respect to the other layers, constitutes the bearing component of the structure of the plantar orthosis, the chassis or frame thereon the other components are assembled.

Such element, with its different geometries and its mechanical characteristics, mainly performs the functions of managing, guiding and distributing on the other components the differentiated deformations of the different foot resting areas during the different phases of the podalic motion.

Then it performs the bearing role of the whole structure of the plantar orthosis 1, and it is the structural basis performing the task of supporting all other structural components and controlling the distribution of the different interactions therebetween.

Then, the base reinforcement layer 5 is the structural element performing the fundamental task of supporting the dynamic motions of the plantar arch in its non-linear action of helical winding/unwinding of pronosupination. Thanks to the particular geometries therewith it is devised and to its intrinsic capability of transferring the motion and the deformations to other structural components of orthosis such base reinforcement layer is capable of generating non-linear deformations.

Generally, but not necessarily, the base reinforcement layer 5 is made of thermoplastic-resin-based material with quite strong stiffness features but further provided with remarkable elastic features, such as, by way of not exhaustive example, known materials with the tradename CrispinFlux^{™}, Superflex^{™}, Rheno^{™}, RhenoFlex^{™}.

It can have thickness comprised between 0.6 mm to 1.2-1.8 mm, depending upon the preferential stiffness and even depending upon the selected level of stiffness degree of the plantar orthosis.

In this way, it is possible obtaining a plantar orthosis which can have from three to five distinct levels of stiffness and mechanical response, other conditions being equal, to support better the physical and functional features of the different users.

By way of example, two tennis players can have the same shoe size but can differ in weight, height, muscle tone and mass, strengths and muscle elasticity, type of physical-athletic features and then, even if they use the same plantar model they will need different resistance, stiffness and elasticity calibrations of the orthosis, which can be obtained by calibrating different thicknesses of the components or by selecting different materials, within a range of possible solutions coherent therebetween.

The plantar orthosis 1 comprises a covering layer 2, or supporting layer, which is the layer in upper position with respect to the other layers and it is the one thereupon the foot rests.

The covering layer 2 has the sizes of the footwear sole base which receives it or incorporates it, and constitutes then the element of maximum extension of the plantar orthosis.

It mainly constitutes the element defining the plantar geometry. For its technical function, it has to perform even the function of micro-dampening of the podalic stresses, but however without allowing the dissipation of the loads and of the forces acting on the plantar rest.

The materials which can be used for the covering layer are then high-density gum-like materials and of not excessively elastic type such as those known with the tradenames EVA^{™}, EVATECH^{™}, EVAELAST^{™}, NORA LUNASOFT^{™} and the like, of traditional use for the resting surfaces of the plantar orthoses.

The thicknesses of the covering layer 2 can vary from 2.5 mm to 4-5 mm, depending upon the comfort level which one wants to implement and, above all in case of very tight sporting footwear such as the soccer ones or the ones for road cycling, upon the available and constraining overall dimensions of the footwear itself.

The covering layer 2 can include a lining covering its upper surface in contact with the plantar rest 3, and which has to protect from wear by rubbing and friction with foot the underneath intermediate components, that is the covering element and discharge supporting elements 6 which will be described hereinafter. Moreover, the lining 3 can perform the task of absorbing and dissipating the foot's sweat, and has to be made of anallergic and bio-compatible materials such as DRYFEET^{™}, Microfibra^{™}, LUXOR^{™}, THUNDER PODODRY^{™}. The thicknesses are the minimum possible ones.

The plantar orthosis 1 comprises a lower layer 4 which performs the task of lower surface finishing, that is the one in contact with the footwear sole, and the one of protecting from wear by rubbing and friction with the sole surface. The lower layer 4 can even perform the task of adhering the plantar to the surface of the footwear sole. Generally, for the lower layer a translucent thermoformable transparent plastic material with a thickness of about 0.7 mm or a finishing material, such as Flexan^{™}, with a thickness of about 0.6 mm, is used.

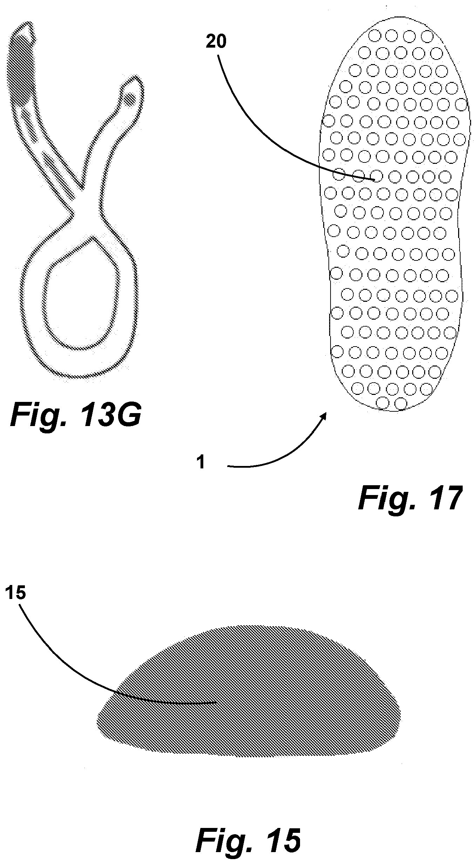

The base reinforcement layer 5 of the plantar orthosis 1 can be optionally coupled to another reinforcing element, called arch reinforcement element 15, that is an additional reinforcement layer which increases the thickness of the base reinforcement layer 5 limitedly to the plantar arch portion of the plantar orthosis 1, joining the heel portion and the portion thereupon the metatarsus rests. Such arch reinforcement element 15, is represented, indicatively, in figure 15 and, if present, is positioned between the base reinforcement layer 5 and the covering layer 2.

The arch reinforcement element 15 is assigned mainly to increase the support of the plantar arch and contributes to the action of the base reinforcement 5 in managing the existing elastic forces, by determining a modification in the thickness of involved cross sections of the plantar orthosis and then of the deformation mechanical properties in such region.

Such modifications of the orthosis mechanical properties in the plantar arch area, obtained with suitable dosage of geometry and thickness of the arch reinforcement 15 are extremely important in producing the suitable management of the linear action of helical winding/unwinding of pronosupination in the podalic helix dynamics during the dynamic motions of the plantar arch.

For this reason, the arch reinforcement layer 15 is generally made of materials based upon thermoplastic resins, with quite marked stiffness features but further provided with strong elastic features, such as for example materials known under the tradenames CrispinFlux^{™}, Superflex^{™}, Rheno^{™}, RhenoFlex^{™}. The thicknesses of such elements can vary from 0.8 mm to 1.4-1.8 mm depending upon the selected material and the stiffness level required with respect to the physical and functional features of the different users, such as for the base layer 2.

The arch reinforcement element 15, by reason of its specific geometric shape and nature of its thicknesses and materials, determines and manages, together with the other structural elements connected thereto, the differentiated deformation of the whole plantar orthosis according to deformation lines coherent with the correct podalic motions defined by complex motion defined by the rolling composition of the resting foot and of the podalic pronation/supination helix.

Above the supporting covering layer 2, the plantar orthosis comprises supporting and discharging elements 6, positioned in the calcaneal region and the metatarsal one, implemented by bearings with various shapes and sizes made of low-density soft gum-like material, such as PORON^{™}, NOENE^{™} and the like, assigned to the discharge and dissipation of the pressure excesses in the most critical areas of the plantar rest. They can be interlocking positioned in suitable recesses 7 or shaped cuttings, performed by means of the cutting plotter on the covering layer. The thicknesses mainly correspond to those of the covering layer 2, but they can even exceed such thicknesses by few mm, so as to be positioned projecting with respect to the surface of the covering layer 2 with the purpose of obtaining even a proprioceptive stimulation of the foot bio-sensors.

Together with said reinforcing and discharging elements 6, the plantar orthosis 1 can include, in its thickness, that is generally on the surface of the covering layer 2 and even by replacing the lining layer 3, a variable number of sensors which can be pressure sensors but even sensors of other nature - as specified hereinafter - 20 (Figure 17), which can be positioned at the heel portion, at the midfoot and at the metatarsal or front portion, or even on the whole surface defined by the covering layer 2.

Moreover, a three-axis accelerometer can be provided, preferably positioned at the plantar arch portion near said arch reinforcement element 15, or even a series of deformation sensors along the whole surface of the orthosis.

These sensors 20 can provide a complex series of detected biomechanical and biological data and be connected to a terminal, such as smartphone or other electronic support, with a wireless connection, for example of BlueTooth^{™} type, whereon a suitable management software is installed, capable of monitoring biological and biomechanics information detected for a complex evaluation of the static and dynamic posture and of other sensitive variables of a user during the real carrying out of the sporting activity.

At last, the plantar orthosis 1 comprises an energy dynamic transport element, or simply dynamic element 8, positioned between the lower layer 4 and the base reinforcement layer 5.

The dynamic element 8 is formed by a belt-like strip, with different shapes and/or sizes, which generally extends from the heel area to the front portion of the plantar orthosis.

In this embodiment example, the dynamic element 8 consists of a belt-like strip which is positioned near the external edge of the plantar orthosis 1 at the heel, and extends with one or more extension strips forming one single piece with the heel strip, so that the dynamic element has a continuity from the heel as far as two portions of front rest of the plantar orthosis 1, positioned near the edge of the plantar orthosis 1, then, with reference to the user's foot sole, one on the external midfoot and the other one along the directrice of the hallux.

In a more preferred embodiment, said belt-like strip, which assumes a bent profile at the heel portion, forms a X-like crossing in a central portion of the plantar orthosis 1, and then it extends with continuity as far as two portions of front rest of the plantar orthosis 1, positioned laterally near the edge of the plantar orthosis 1, that is at the first and of the fifth metatarsus, respectively.

The dynamic element 8 contributes with its own thickness, together with the reinforcement layer di base 5 and in case the arch reinforcement 15, if present, to generate the non-linear deformation of the plantar orthosis during the foot resting phase and to implement the targeted deformation of the orthosis according to the privileged directrices detected by the biomechanism of the podalic helix.

The dynamic element 8 is made of elastic material which is deformable, thanks to its elastic features, to a greater extent than the layers enclosing it.

Therefore, the dynamic element 8 is further the element assigned to define the privileged directrices of the elastic potential energy transfer during the motion of unwinding and winding of the podalic helix, as above described, by managing the privileged directrices of elastic wave propagation during the deformation of the plantar surface in the podalic motion. This element 8 interacts, in such activity, with the base reinforcement layer 5 and with the arch reinforcement element 15, in case it is possibly present, in the phase for accumulating, distributing and releasing the elastic energy generated inside the orthosis during the deambulation motion and it constitutes the main element in the address of such energy according to predetermined directrices.

The dynamic element 8, together with the base reinforcement layer 5 and to the arch reinforcement element 15, then is the structural element capable of deforming elastically, by lengthening or shortening, in different directions or being subjected to twisting undergoing the action of forces applied thereto and afterwards going back to its own natural shape; its elastic properties allow it to be advantageously compressed by a force applied on its surface or subjected to traction. Its main purpose is to accumulate, address, transfer and release the mechanical energy which has developed on the whole plantar orthosis.

The same material thereof the dynamic element 8 is made, is capable of varying its own thickness and, if it is subjected to pressure, deforms elastically and it is capable of generating inside thereof a pressure elastic wave which transmits along its length and afterwards going back to its natural shape. Its elastic capabilities, that is its elastic modules according to various directions, could be linear or preferably non-linear.

The base thicknesses of the dynamic element 8 can vary from 1.0 mm to 1.4-2.2 mm according to the selected material and the elastic stiffness level required with respect to the physical and functional features of the different users.

The dynamic element 8, together with the base element 5 and the arch reinforcement 15, then is a structural element capable of deforming elastically, by lengthening or shortening, in different directions or being subjected to twisting by undergoing the action of forces applied thereto with the purpose of managing the privileged directions of elastic wave propagation.

Such dynamic element 8 is capable of generating inside thereof an elastic wave and, afterwards, of going back to its own natural shape; its purpose is to accumulate, transfer and return mechanical energy. It is an element of elastic type the elastic capabilities thereof (elastic modules according to various directions) could be linear or preferably non-linear: advantageously its elastic properties allow it to be compressed by a force applied on its surface or subjected to traction.

The dynamic element 8 can be manufactured in different materials, such as plastics, polymers and composite materials with glass fibres, carbon, Kewlar^{™} and other aramid fibres, metals and alloys such as steel, titanium, tungsten, tantalum. To this purpose shapes could be devised, for such structural elements, recalling all types and principles of the springs known in mechanics according to the use and of the functions thereto such components are assigned in the design phase.

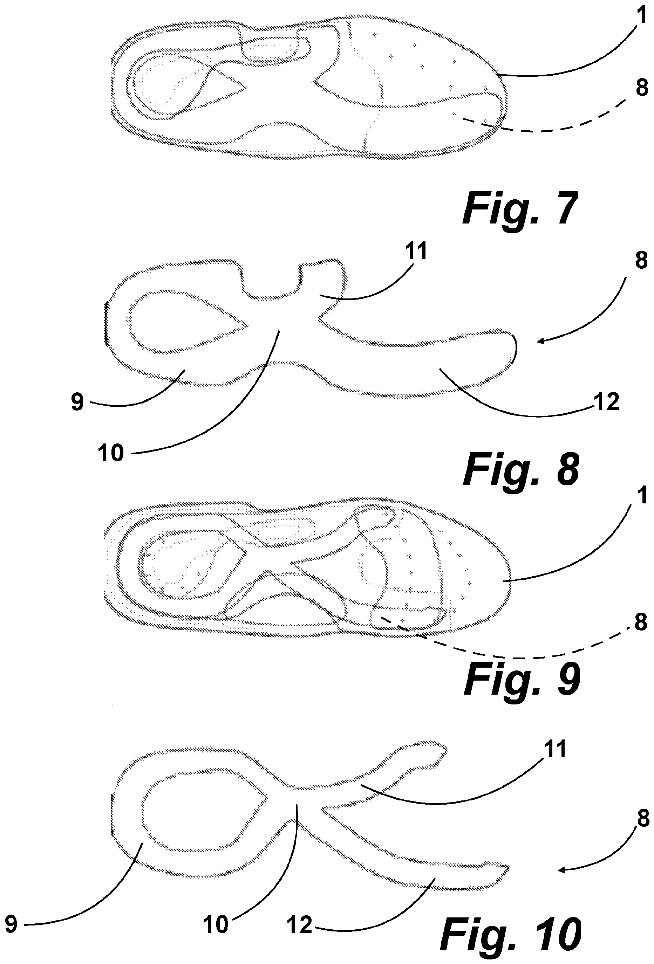

With reference to the images from Figure 7 to Figure 10, two embodiment examples of plantar orthosis 1 are shown, both with an inner dynamic element 8 formed by a belt-like strip which, at the heel portion of the plantar orthosis 1, forms a ring 9 arranged near the edges of said heel portion.

In the plantar arch portion of the plantar orthosis 1, the dynamic element 8 joins by forming a X-like crossing, and subsequently it extends with a first internal branch 11, towards the fifth metatarsus, and with a second external branch 12, in direction of the first metatarsus, that is of the hallux, with an end extending substantially along the whole first metatarsus.

With reference to figure 8, the internal branch 11 of this first embodiment example extends as far as overcoming the border between heel and metatarsus, and has one terminal end near such border.

With reference to figure 10, a second embodiment example is shown, the internal branch 11 extends for the whole metatarsal bone of the fifth metatarsus.

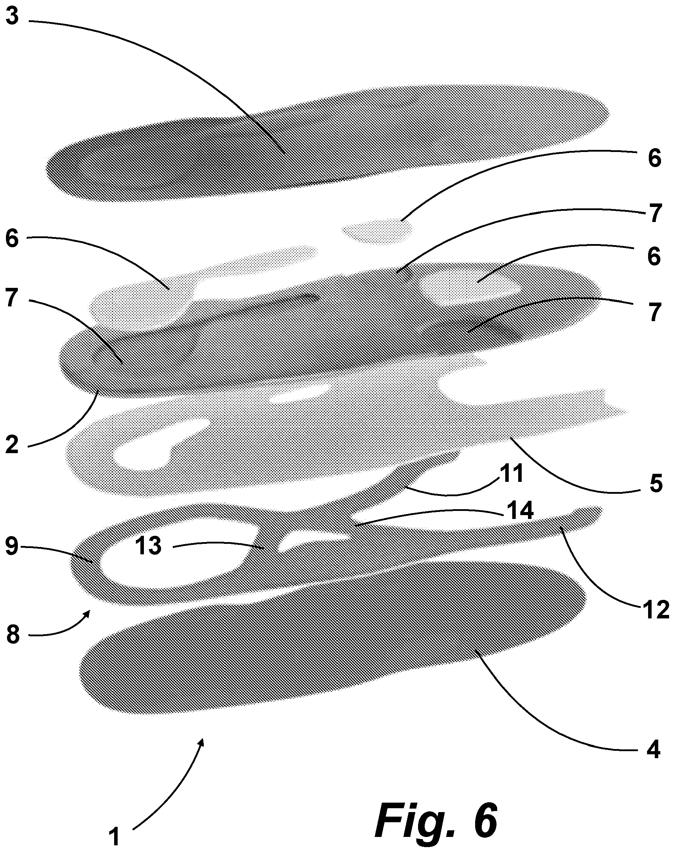

With reference instead to figure 6, a dynamic element 8 with a different junction between ring 9 and branches 11 and 12 is shown: the ring 9 is closed again by a first cross branch 13 whereas, laterally, it extends parallelly to the edge of the plantar orthosis 1, by joining to the internal and external branches 11 and 12 which are further joined by a second cross branch 14 forming, with the first cross branch 13 and with the initial tract of the internal and external branches 11 and 12 comprised between both cross branches 13 and 14, a quadrilateral, the position thereof substantially corresponds to the plantar arch portion of the plantar orthosis 1.

These three embodiment examples, and all variants comprised and not thereamong, considering apart from shape changes also changes in materials and thicknesses, can be adapted to several activities, thus allowing to plan a plantar orthosis gradually suitable to a specific activity, which could be sporting, military activity or other specific activity or referred to the daily activity of users which require posture corrections or antalgic actions.

Moreover, it is to be meant that the above-mentioned plantar orthosis, comprising the mentioned dynamic elements and relative specific functions, could be applied to any foot-ground interface such as, by way of example, footwear soles, socks, inner soles, orthopaedic plantar insoles, insoles integrated in footwear and so on.

Thanks to the above-described dynamic element 8, reinforcement layer di base 5 and, in case, reinforcement layer di volta 15 and to the thicknesses differentiated therefrom generated in its cross sections, the plantar orthosis 1 has the capability of deforming, during the podalic resting phase in motion, in differentiated and preordinated way, in particular by following the directrices defined by the foot's own motion and of transferring elastic energy in suitable defined directions.

The complex motion of the foot during resting is the composition of its rolling motion in the motion direction and in the direction of the motions dictated by the podalic transmission bio-mechanism, which translate into the so-called winding and unwinding of the podalic helix. This complex motion determines a continuous modification in the foot architecture during the motion development. By simplifying, apart from the natural rolling motion in the forwarding direction there are the pronation and supination motions due to the podalic helix motion in phylogravitatory (unwinding) phase and antigravitatory (winding) phase which are synchronous and alternated between the backfoot (heel) and forefoot (metatarsus), which correspond to the portion of the heel and to the front portion of the orthosis 1.

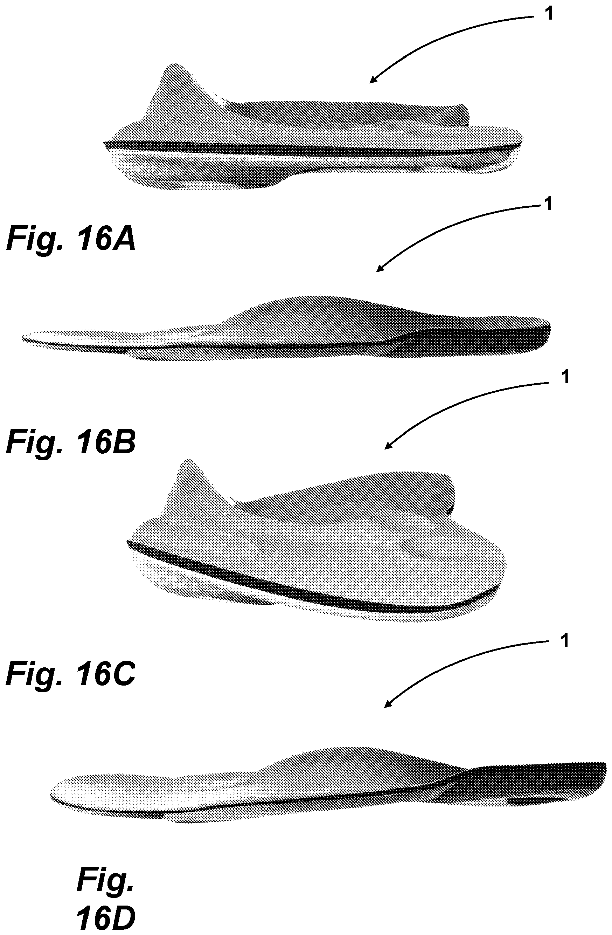

Such deformations of the plantar orthosis 1 predetermined by the planning in agreement to the dynamics of the podalic helix transmission bio-mechanism are described in the images contained from Figure 16A to Figure 16G.

Through such technical solution, the natural helix motion of the foot is supported, in case of absence of postural defects of the subject in deambulatory phase, and guided in case of presence of such postural defects. Since a postural defect of the plantar rest determines automatically a defect in the muscle and articular activation in all body segments interconnected with the foot, according to the respective kinematic chains connected thereto, it is understood that this can generate the appearance of operation unbalances, and then injuries in such segments under critical conditions of physical stress.

The dynamic element 8 and the base reinforcement layer 5, in case together with the arch reinforcement 15, during the resting phase are compressed and deformed during the foot unwinding motion, and extend and tend to go back in their original shape during the winding phase: by doing so they transmit energy through an elastic deformation which is not homogeneous due to the different thicknesses and the consistencies differentiated in the various cross sections of the orthosis itself by collecting it from resting areas in which they could result to be dangerous and by releasing them, thanks to the action of the dynamic element 8, in different areas in which they can constitute an advantageous support and stimulus to the involved podalic forces to the positive and correct phase of the motion by favouring, in this way, the correct motor activations even in the ascending segments of the whole lower limb and of the trunk belonging to the involved kinematic chain.

The non-linear deformation of orthosis, managed by the inhomogeneity in consistency and thicknesses of the cross sections, is then capable of following the natural composite motion of the foot and of favouring the address and distribution of the energies accumulated in the plantar orthoses 1 according to the physiological lines defined by the correct motion of the foot in its different dynamic configurations.

In detail, it is possible to schematize in first approximation the foot motion its phase for resting on the ground as non-linear composition, of its rolling motion, wherein the foot develops an arching bending of its structure along its longitudinal axis, with the two winding and unwinding motions of the podalic helix biomechanics. Just as schematically it is possible to divide such overall foot motion during resting in phases: 1. Contact, 2. Intermediate phase evolving in some sub-phases, and 3. Push.

In the first contact phase, the foot starts resting on the ground and keeps a neutral asset with respect to its axes; the plantar orthosis remains neutral with respect to its deformations on the three axes (Figures 16A and 16B).

In the subsequent Intermediate phase of the contact with the ground, a first sub-phase is characterized by keeping the foot neutrality with respect to its longitudinal axis, but even by the presence of a pronation motion of the heel and a consequent supination motion of the forefoot, determined by the triggering of the unwinding dynamics of the helix biomechanics. In this phase, the insole supports and manages such motion through a helix-like twisting guided deformation along the longitudinal axis (Figures 16C and 16D), favourable to heel pronation and forefoot supination.

In its deformation, the plantar orthosis accompanies such motions and manages the intensity thereof, by even adjusting the suitable widths.

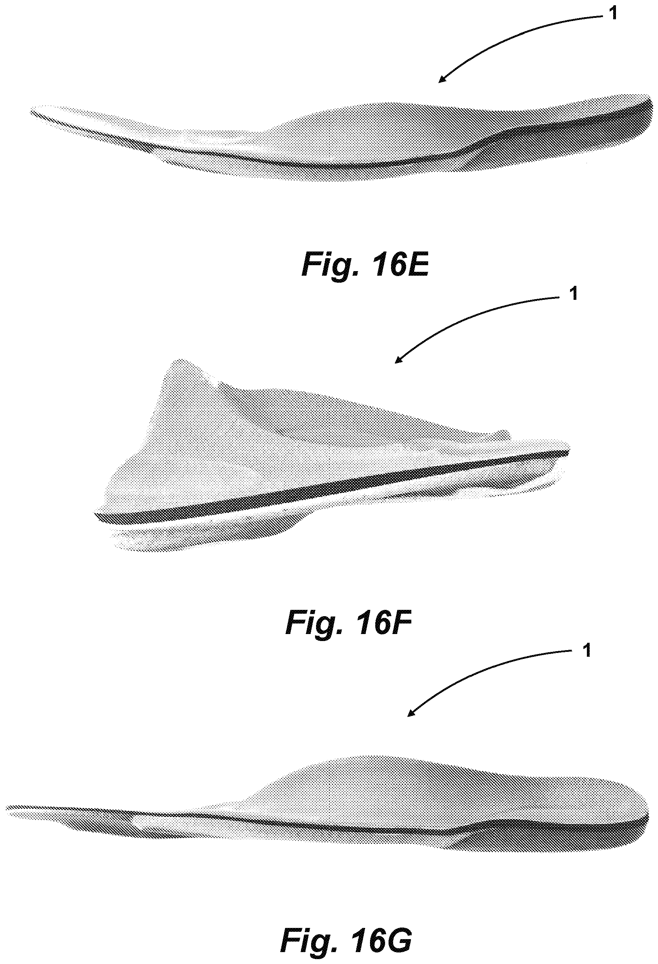

In the subsequent evolution of the Intermediate phase, a second sub-phase is carried out in which the motions of heel pronation and forefoot supination regress towards neutrality of the podalic asset, and at the same time the foot bending and rolling dynamics along the directrice of its longitudinal axis evolves and takes over. In such sub-phase, the insole, in agreement with the podalic dynamics, reduces progressively its own twisting deformations along the longitudinal axis and recovers neutrality with respect to such deformation directrices. In agreement with the foot motion, the plantar orthosis develops progressively a bending deformation along the longitudinal axis (Figure 16E) and once again supports, guides and assists the foot motion.

In the subsequent third resting phase, the Pushing one, the podalic helix biomechanics imposes the contraction of the foot structure, the podalic helix winding and the consequent heel supination and forefoot pronation to generate the push; apart from such dynamics firstly an additional bending takes place and then its progressive inversion, in the extension of the plantar bending which had generated in the preceding phase for the foot rolling motion along the directrice of the longitudinal axis. In such phase, the plantar orthosis activates a helix-like twisting controlled deformation along the longitudinal axis (Figures 16F and 16G) favourable to the heel supination and to the forefoot pronation and subsequently recovers progressively neutrality with respect to the bending deformation along the longitudinal axis. In this new deformation, the plantar orthosis still accompanies the foot torsional motions and manages the intensity thereof by adjusting even the suitable widths.

### EXAMPLE

In order to illustrate the operation of the above-mentioned plantar orthosis, an explanatory example is reported herebelow.

During running four phases can be detected which constitute the working cycle of the lower limbs.

The first phase is the flying one: once the bearing limb has detached from the ground, the limbs, with the fulcrum at the pelvis, act in opposite direction; the foot of the free limb moves quickly forward due to the effect of the thigh and leg extension. The foot of the pushing limb moves away from the pelvis; the knee joint results to be almost wholly extended and the hip is directed backwards.

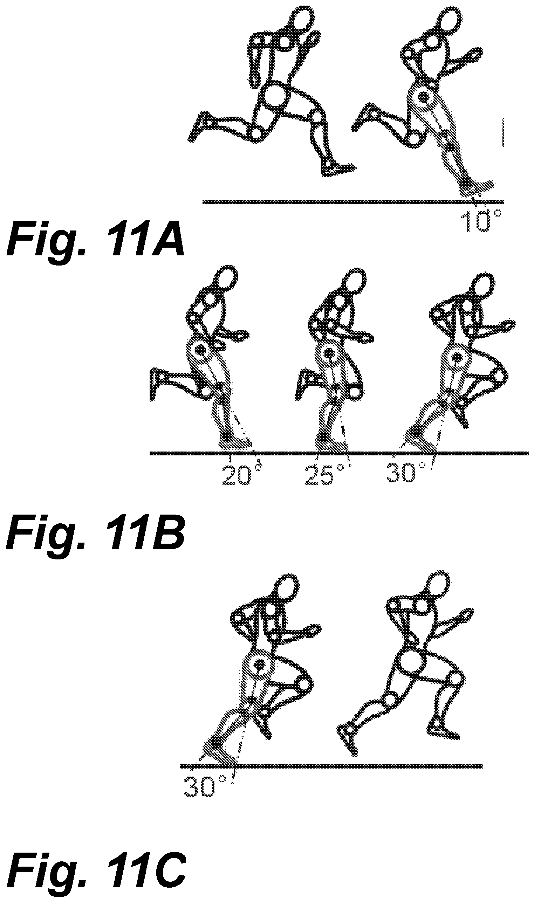

Whereas the rear limb moves forward, the foot of the front one falls downwards just in front of the knee. The limb lowering towards the ground allows the foot to assume the resting position nearest to the pelvis vertical (Figure 11A).

The arrival on the ground follows, that is the establishment of contact with the ground which takes place in a first time with the foot external portion, and then the resting surface increases until the body passes thereabove: the lower limb yields, by bending, upon the impact; in this moment the athlete settles the grip to the ground of the foot by dampening the centre-of-gravity motion downwards with consequent slowing down of the horizontal speed (Figure 11B).

Once ended the dampening phase, the pushing phase starts, that is the positive portion of the foot action with the application of the force on the ground and the relaxation of the leg extensor muscles. The extending phase starts from the pelvis muscles, slower but powerful, to end up with the foot muscles; the action completes with the foot far from the pelvis, which leaves the ground with its internal portion (Figure 11C).

The most important phase, with the purpose of better understanding the injuries due to running, is the resting phase which can be divided into three periods:
Contact: the foot has relationship with the ground with the external edge, due to the foot inversion at time of impact. This part lasts 25% of the total resting time. In this phase, the resting foot is in front of the body centre of gravity whereas the forefoot gets in contact with the ground and dampens the impact even thanks to the action of the triceps sural. To say the truth the runners make contact with the ground in three ways, with forefoot, with heel or with the whole foot sole.
Intermediate: during this period, which corresponds to 40% of the resting time, the foot is in eversion. Pronation, in fact, is a normal foot motion which contributes in mitigating the impact with the ground, by transmitting it from the heel to the foot remaining portion. The foot pronation is a passive event, caused by the overload of the body weight on the lower limbs and on the subtalar joint, which in contact with the ground favours pronation for the configuration itself of the joint.

In particular, under the body weight the talus and heel adapt progressively on the subtalar joint surface of the foot with reverse motion therebetween: the talus supinates, whereas the heel pronates towards inside.

This motion then transmits in mid-tarsal seat of the foot with the helical motion biomechanism by causing the winding thereof, that is the mid-tarsal area of the foot behaves like a helical spring which under the action of stresses unwinds, that is it lengthens. In this phase, the dynamic element 8 and the reinforcement layer 5 and in case the reinforcement 15 produce an action which limits the levelling of the foot longitudinal arch.

Push: the foot in this phase has a supination motion; due to the muscle action and the anatomical ratios of the tarsal bones it "stiffens", it accentuates the longitudinal arch and the heel goes in eversion supination. The heel, under the action of the triceps sural, supinates whereas talus, locked by the malleolar forceps allows the rotation of the heel itself on its lower surface and allows the motion transmission to the foot midtarsal tract which determines the biomechanism with helical motion which develops in a winding motion, that is the foot midtarsal tract behaves like a helical spring which winds, that is contracts.

The propulsive phase takes 35% of the resting time. During the resting median phase the subtalar eversion is accompanied by the inner rotation of tibia and fibula. The rotula, for its position in the femur intercondylar throat follows the motions of the femur itself.

By analysing in detail the biomechanical features, and by keeping into consideration the muscles which work during this period, it happens that, in the dampening phase, the resting foot is in front of the body centre of gravity; in this case the forefoot generally takes the first contact with the ground, but most part of the impact dampening action takes place with the foot pronation action, starting from the heel, above all thanks to the triceps sural action.

During the supporting phase, the foot is perfectly in axis with the centre of gravity; the muscles contract isometrically to favour the body stability. The quadriceps, for example, contracts to brake the body fall: this type of contraction however takes place with a lengthening of the muscle belly, and in fact a leg bending occurs. In this case one speaks about eccentric contraction, that is the muscle, even if it lengthens, develops tension.

In the pushing phase the foot is behind the centre of gravity and prepares to push by stiffening: the muscles exploit their force of elastic and reactive type to project forward the body, then the leg relaxation takes place. The pushing phase starts from the pelvis muscles, slower but powerful, it continues with the leg's muscles and ends up with the foot muscles. The rear muscles of the thigh come into play during the knee forwarding, to avoid hyperextension of the leg: it is an eccentric contraction which protects the muscles themselves which otherwise would risk to stretch. A fast forwarding of the leg, kicked forward, if it is not limited and slowed down, causes trauma.

The deep muscle of the calf, called soleo, is the one that, together with the gluteus maximus, provides the most important contribution for the motion genesis. The contribution of the rectus femoris instead is low and the quadriceps, as a whole, assumes a certain importance only in preserving the joint from the foot impact on the ground.

The muscles of the pelvis and of the trunk act as stabilizers during the whole motion (rectum of the abdomen, internal oblique muscles, external oblique muscles, sacrospinal or iliocostal and longissimus muscle of the back, quadratus lumborum muscle and large dorsal muscle).

As far as the case study related to the most recurrent injuries in running is concerned, by leaving out the even very widespread and incident inflammatory phenomena apart from the permanent damages generated by wear, one finds that most part is detected among those of muscle type going from contractures to muscle lesions with different gravity degree (from stretching to tear). The most affected tendon part is Achille's tendon whereas for the muscle part the most affected ones are the gluteus medius, the biceps femoris, the quadriceps and the calf.

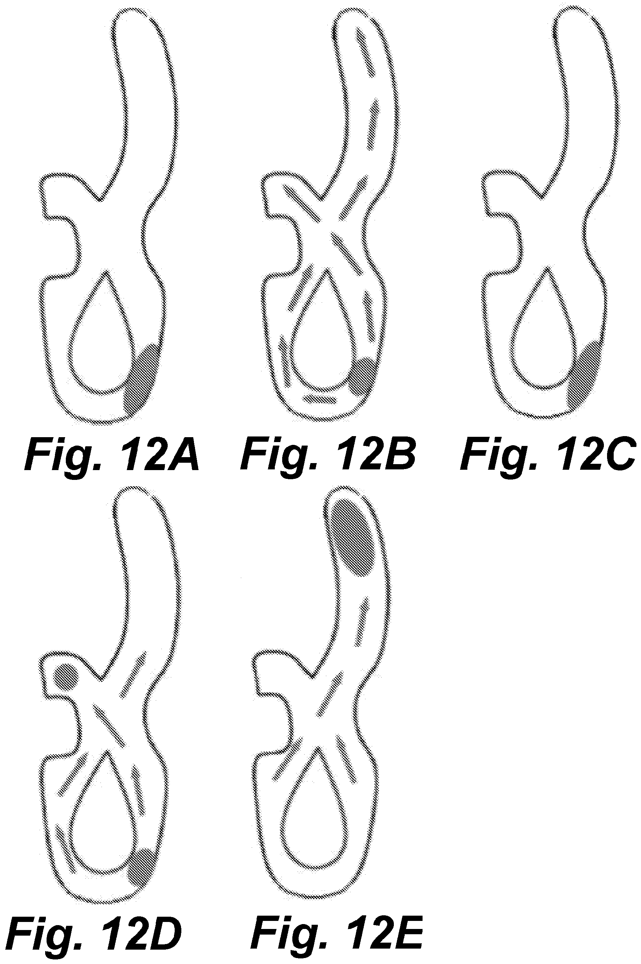

With reference to figures 12A to 12E, it is possible to describe the way in which the plantar orthosis 1 allows to manage the different phases of running by providing a dynamic contribution to each one thereof and by allowing a decrease in the maximum stresses, above all in the most impulsive cases.

In the contact phase, the foot impact with the ground takes place starting from the forefoot until involving its totality. The muscles which activate are the calf triceps sural, the biceps femoris and the quadriceps all in eccentric phase. In the intermediate phase, the foot starts to manage the impact dampening through pronation starting from the heel and then involving the mid-tarsal portion in phylogravitational phase. The active muscles in this phase are still the calf triceps sural, the biceps and the quadriceps femoris.

The plantar orthosis, in this first phase, above all manages the absorption of the kinetic energy through its transformation into elastic potential energy against the dynamic element 8 and the base reinforcement layer 5 (Figure 12A), with its transfer along preferred directrices defined by the shape of the dynamic element 8 (Figure 12B).

The plantar orthosis, as a whole, supports pronation through its elastic action, by sustaining it and controlling that it remains in a suitable range, whereas the dynamic elements continue to accumulate potential energy.

While the body moves forward, by varying the position of the centre of gravity from behind the resting foot to forward, the dynamic element 8 and the base reinforcement layer 5, by following and supporting the progress of the unwound-helix motion of the mid-tarsus, continue to store elastic potential energy and in the same time the dynamic element 8 performs the migration of components of such energy along its directrices.

In the pushing phase, the foot starts stiffening, accentuates the longitudinal arch and in the heel the inversion from pronated position to supination one takes place. The heel, under the action of the triceps sural, supinates and the motion, through the talus pronation, transmits to the mid-tarsal tract of the foot that determines the biomechanism with helical motion which develops in a winding motion. In such phase the foot resting starts moving more and more on the front, in a first moment towards the external area (fourth and fifth metatarsus - Figure 12D) and then in the internal front portion (first, second, third metatarsus, distal portion and hallux - Figure 12E).

The muscles which activate to generate the push are those of the gluteus and the calf triceps sural, all in concentric phase, whereas the thigh flexor works as antagonist in the motion.

The plantar orthosis, in this phase, release in the calcaneal area part of the stored elastic potential energy by favouring the heel inversion from pronated to supinated (Figure 12C) and, after having completed through the dynamic element (8) the transfer of other component of such elastic energy in the forefoot area (Figure 12D) it releases it as elastic kinetic energy to favour the pushing phase thus by helping the action of the triceps sural at first and then of the gluteus muscle (Figure 12E).

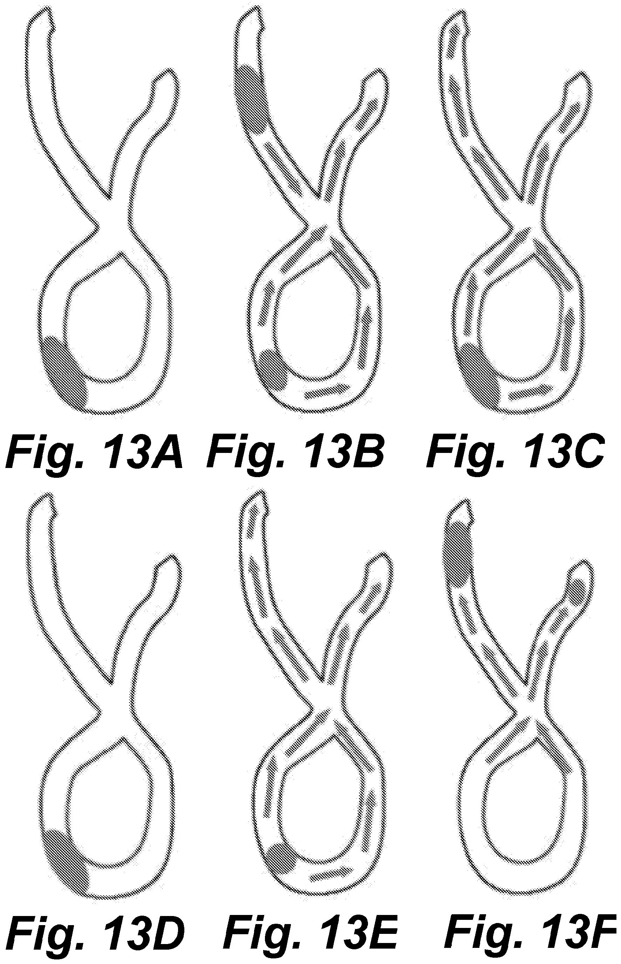

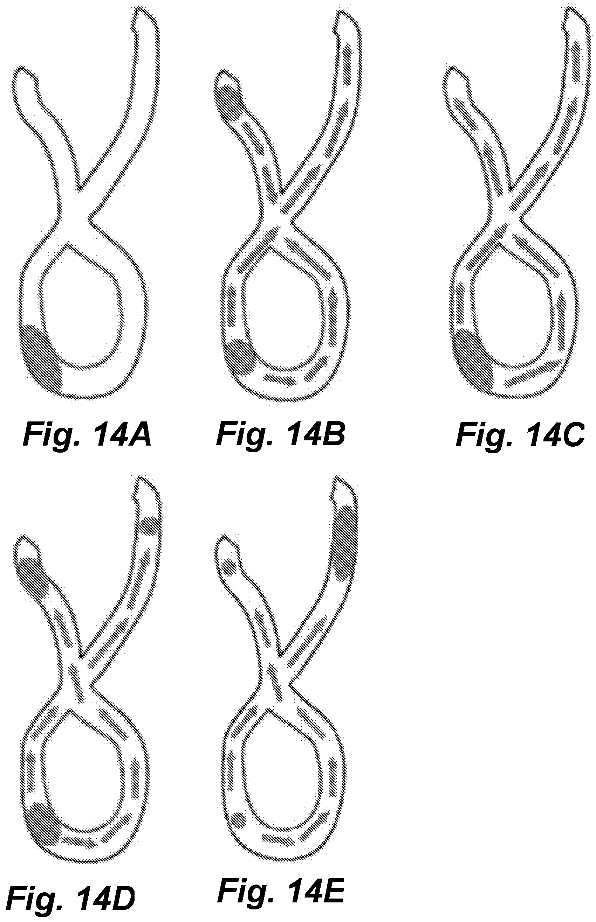

With reference to figures 13A to 13F and 14A to 14E an effective change in direction will be described hereinafter, which is characterized by five fundamental phases:
1) penultimate step (deceleration), is the moment in which the brake starts to face the change in direction (faced by the internal limb with respect to the change in direction)
2) last step (deceleration) is faced with the limb opposite to the previous one (external leg), in this case the brake is accentuated.
3) change phase, which is faced with the limb opposite to the phase of the last step (internal limb); it is the most important moment of the gesture, in this phase first of all one continues to decrease speed, then the twisting is performed to address the body towards the new running line and at last the propulsion phase starts with the foot push on the ground.
4) first step is performed with the same limb of the last step (external limb); this limb has the function of the pin whereon the body rotates, then afterwards the same limb performs an important push for starting again the running itself;
5) flight phase starting again running (no foot remains rested onto the ground).

In different sports, the change in direction is repeated with high frequency; for it quick decelerations and accelerations are required; in particular among such motion components in literature it seems it can be assumed that the effects of the deceleration component determine an increase in the potential for injuries.

Each phase is then examined in detail.

In phases 1 and 2 (penultimate and last step), both limbs are engaged in a front deceleration wherein all joints (ankle, knee and hip) are in eccentric phase. The most stressed components are the muscle and tendon ones destined to slow down; in particular Achille's tendon, the triceps sural and the quadriceps femoris.

In phase 3 (change) the change in direction is implemented and, together with the subsequent phase, it is the most critical phase. In it, the internal limb is engaged in the motion inversion and then has the greatest moments and the greatest rotations on the different axes.

The knee has a wide rotation on the abduction-adduction plane. The powers developed on all joints are eccentric but, during the motion, they reverse and become concentric. Then, the knee is in a situation of extreme stress between the opposed actions of the two adjacent joints, hip and ankle.

In this phase, the ankle has rotations on the front plane, but it does suffer a rotation on the abduction-adduction plane: the rotary stresses existing on the ankle on the abduction-adduction plane are discharged on the talus (pronation) and consequently on the heel (supination) through the foot transmission bio-mechanism. The rotary tensions on the cross plane constitute then the maximum risk for the ligaments of the knee joint, and the maximum rotations on the front plane constitute the greatest danger for the ankle integrity.

In phase 4, the external limb is mostly engaged in the deceleration and acceleration phases; during it, then, the highest stresses in intensity appear with respect to the internal limb and of more impulsive type since the maxima develop in shorter time, but the greatest rotations are those on the flexo-extension plant for all articular joints, that is ankle, knee, hip. The courses of the angular moments of the limb's articular joints generally are not correlated. At first, the most stressed joint is the ankle, since it has angular displacements on all three axes, and the maximum angular peak develops at the same time under the maximum rotation condition (talus in supination and heel in pronation) and maximum flexo-extension.

Moreover, at the instant of beginning the motion inversion, both the hip and the knee develop concentric powers and then the ankle articular has to work in eccentric phase, by absorbing the generated power of the other two articulations, and this happens in the precise moment wherein the hip develops its maximum of concentric power. The ankle then is put in the situation of maximum stress.

The ligaments, in this context the lateral ones, are the anatomical elements most at risk. Subsequently, the ankle generates again concentric powers, by inducing the inversion on the knee which, in turn, develops again eccentric power and then it has to absorb the power generated by the other two articular joints. Therefore, the maximum stresses now reach the knee articulation which, even if the most important rotations are localized on the flexo-extension plane, also have rotations on the cross plane. Then, in this motion phase, the cruciate and latera collateral ligaments are more at risk of lesion.

In the subsequent phases, at last, both limbs are engaged in an acceleration, with situations very similar to those of phases 1 and 2, but with often inverted stress directions. In fact, both limbs are engaged in an acceleration, and all articular joints (ankle, knee and hip) are in concentric phase. The most stressed anatomical components are the muscle and tender ones, in particular Achille's tendon, the triceps sural and the gluteus.

The most important phases, from the point of view of the probability of occurrence of injury and then of greater interest, are evidently phase 3 and phase 4, which will be described in detail hereinafter.

In phase 3, related to the so-called change, the foot inside the motion has contact with the ground, mainly with the backfoot and with the external edge of the foot in eversion with the pronated talus and the supinated heel. The foot, then, is under conditions of maximum contraction having the podalic helix in winding position. In order to support the dampening of impact, which in this phase mainly burdens on the medial ligaments of the ankle, and to discharge such stresses on the triceps sural the podalic helix has to unwind by favouring inversion of podalic eversion and make the talus and the heel to evolve towards the neutral position from their initial respective pronation and supination. Therefore, the helix winding, allowing the extension and controlled relaxation of the foot, reduces so as to manage dampening upon impact.

Subsequently, in the need for generating the propulsive push, the foot stiffens again under the action at first of the posterior tibialis and peroneal longus, and then of the triceps sural, which forces the talus and heel to bring them respectively back to the initial position respectively of pronation and supination.

The push to rotation of talus allows the motion transmission to the foot mid-tarsal tract which determines the bio-mechanism with helical motion which develops in a winding motion and generates the push. The foot resting pressure starts moving towards the front area starting from the external one (fourth and fifth metatarsus) tending towards the internal one (first, second, third metatarsus) and then towards the distal portion and the hallux.

In phase 4, related to the so-called first step, the foot outside the motion has contact on the ground with the medial front portion and subsequently with the whole foot sole. In the first phase, the one of the impact dampening, the foot is already in strong inversion, and the talus and heel, in attempting to adapt progressively to the ground, try to further supinate the talus, and to accentuate the heel pronation, and to transfer the motion energy in mid-tarsal seat of the foot with the helical motion bio-mechanism with a winding.

For the position already rotated on the front plane of the ankle, the ones strongly at risk in such phase - since they are close to their limit in extension, are its lateral ligaments. Subsequently, in the need for generating the push, the concentric phase of the triceps sural starts which tends to bring the heel in supination, and consequently the talus in pronation.

The talus rotation transmits the motion to the foot mid-tarsal tract wherein the bio-mechanism with helical motion in winding is triggered by starting the propulsive phase. The foot resting pressure starts to move towards the front area (more in the internal one of the first, second, third metatarsus) and then towards the distal portion and the hallux.

In the light of what described above, the behaviour of the plantar orthosis is detailed hereinafter with particular reference to the above-described phases 3 and 4 and to figures 13A to 13G and 14A to 14E.

With reference to the change phase 3, a first sub-phase is provided wherein the foot inside the motion has contact with the ground, mainly with the backfoot and the external edge in eversion with the pronated talus and the supinated heel. The foot, through the helix biomechanics unwinds the podalic helix by extending and managing the dampening upon impact. The plantar orthosis contributes in absorbing the impact kinetic energy through its transformation into potential energy and storage of the same in the dynamic element and in the base reinforcement layer 5 (figure 13A). Subsequently, by following and supporting the progress of the helix unwinding motion of mid-tarsus, they continue to store elastic potential energy and at the same time the dynamic element 8 performs the migration of components of such energy along the directrices defined by the arrangement of the continuous belt-shaped strips thereof it consists, and by the dynamics of the podalic unwinding (Figure 13B).

In the same change phase, a second sub-phase is provided: the foot starts its stiffening to generate the propulsive push, and the triceps sural starts exerting its action on the heel and talus, by making them to recover their initial position with respective supination and pronation. In this phase, the tension maximum develops of the ankle medial ligament which is in the maximum of its extension. The plantar orthosis then, with its elastic action, manages the foot inversion action towards the heel supinated initial position by sustaining the area in supination and continuing to absorb elastic potential energy (Figure 13C).

In a third sub-phase, the talus rotation (in pronation), consequent to the heel supination, allows the motion transmission to the foot mid-tarsal tract which determines the biomechanism with helical motion which develops in a winding motion and generates the push. The foot resting pressure starts moving towards the front area starting from the external one (fourth and fifth metatarsus) tending towards the internal one (first, second, third metatarsus) and then towards the distal portion and the hallux. The plantar orthosis, in this sub-phase, proceeds with returning the potential energy accumulated in form of elastic kinetic energy by favouring the pushing phase and the action of the triceps sural, starting from the calcaneal foot dorsal (heel, cuboid, fourth and fifth metatarsus and relative distal portion) (Figure 13D), and then tending towards the internal one (first, second, third metatarsus) (Figure 13E) and then towards the distal portion (Figure 13F) and the hallux (Figure 13G).

Turning now to the phase of first step 4, a fourth sub-phase is provided wherein the foot outside the motion has contact on the ground with the medial front portion, and subsequently with the whole foot sole. The foot is already in strong inversion and the talus and the heel, in attempting to adapt progressively to the ground, tend both to further supinate the talus and to accentuate the heel pronation, and then to transfer the motion energy in foot mid-tarsal seat with the helical motion bio-mechanism, with a winding. The plantar orthosis here manages and accompanies such foot inversion with an elastic contribution which contributes to absorb the impact kinetic energy through its transformation into potential energy and storage of the same inside the dynamic element 8 and the base layer 5 (Figure 14A).

By following and supporting the progress of unwinding motion of the mid-tarsus helix, the two involved elements, the dynamic element and the base reinforcement layer 5, continue to store elastic potential energy and, at the same time, the dynamic element 8 performs the migration of components of such energy along the directrices defined by the element 8 itself and by the helix unwinding motion (Figure 14B and 14C).

In a fifth sub-phase, the foot starts its stiffening to generate the propulsive push: the triceps sural starts to exert its action on the talus, the heel supinates whereas the talus pronates. The plantar orthosis then supports the action of heel supination and talus pronation by returning previously stored potential energy (Figure 14D).

In a sixth and last sub-phase, the talus pronation rotation transmits the motion to the foot mid-tarsal tract where the biomechanism with helix motion in winding is triggered: the foot stiffens and the propulsive phase develops. The foot resting pressure starts moving towards the metatarsal front area, mainly the internal one of the first second and third metatarsus; and then towards the distal portion and the hallux. The plantar orthosis, after having completed the elastic energy transfer to the forefoot area, releases it as elastic kinetic energy to favour the pushing phase (Figure 14E).

In conclusion, the above-described plantar orthosis can act by rebalancing the posture of his/her user, and favours the increase in the force and coordination of the muscles, by allowing their activation according to their correct sequence.

Moreover, the control and management of the directions of reaction forces are favoured, with the stabilization of the articular joints and normalization of the functional unbalances of the muscle kinematic chains.

Moreover, a decrease and normalization of the dynamic load peaks on the muscle and joint apparatus of the whole lower limb are facilitated, thus a decrease in the risk of muscle and joint injuries, due to impulsive and/or overload phenomena, is obtained.

To the above-described plantar orthosis a person skilled in the art, with the purpose of satisfying additional and contingent needs, could bring several additional modifications and variants, all however comprised within the protective scope of the present invention, as defined by the enclosed claims.

## Claims

1. A plantar orthosis (1), for supporting a user's plantar arch, wherein a heel portion and a metatarsus portion are identified, separated by an intermediate portion of plantar arch, comprising:
• a covering layer (2) intended to receive a user's resting foot;
• a finishing lower layer (4);
• a base reinforcement layer (5), intermediate between said covering layer (2) and lower layer (4), supporting and guiding the dynamic motions of compression, extending and twisting the plantar arch in the non-linear action thereof of helical winding/unwinding of pronosupination;
• a dynamic element (8), positioned between the base reinforcement layer (5) and the lower layer (4), which is formed by a continuous belt-like strip, arranged near the external edge of the heel portion, and which extends with one or more belt-like branches as far as respective front resting portions of the plantar orthosis (1) at the external midfoot and near the hallux of said plantar arch,
wherein the continuous dynamic element (8) is made of elastic material which is deformable to a greater extent than both the base reinforcement layer (5) and the lower layer (4) including it, and wherein said continuous belt-like strip elastically deforms and varies its own dimensions in the different directions, as a result of said non-linear action of helical winding/unwinding of pronosupination, by generating thereinside a pressure elastic wave along its length and later returning to its own natural shape, so as to store, transfer, address and release elastic mechanical energy from the heel portion to the metatarsus portion and vice-versa according to preferential privileged directrices.

2. The plantar orthosis (1) according to claim 1, wherein said base reinforcement layer (5) is coupled to a structural arch reinforcement element (15), increasing the the base reinforcement layer (5) thickness limitedly to the plantar arch portion of the plantar orthosis (1), joining the heel portion and the portion thereupon the metatarsus rests.

3. The plantar orthosis (1) according to claim 2, wherein said arch reinforcement element (15) is positioned between the base reinforcement layer (5) and the covering layer (2).

4. The plantar orthosis (1) according to claim 3, wherein the arch reinforcement element (15) is coupled to the base reinforcement layer (5) and to the dynamic element (8) so as to determine, along with them, a differentiated deformation of the whole plantar orthosis according to deformation lines coherent with the correct podalic motions defined by complex motion defined by the rolling composition of the resting foot and of the podalic pronation/supination helix.

5. The plantar orthosis (1) according to claim 1, wherein said belt-like strip, at the heel portion of the plantar orthosis (1), forms a ring (9) arranged close to the edges of said heel portion.

6. The plantar orthosis (1) according to claim 5, wherein the dynamic element (8) joins by forming, at the plantar arch portion, a X-like crossing (10) therefrom a first internal branch (11) extends towards the fifth metatarsus, and, a second external branch (12) extends towards the forst metatarsus of the user's foot, with an end extending substantially along the whole first metatarsus.

7. The plantar orthosis (1) according to claim 6, wherein the internal branch (11) extends as far as overcoming the border between heel and metatarsus of the user's foot, and has one terminal end near such border.

8. The plantar orthosis (1) according to claim 6, wherein the internal branch (11) extends for the whole metatarsal bone of the fifth metatarsus of the user's foot.

9. The plantar orthosis (1) according to claim 6, wherein the ring (9) is closed again by a first cross branch (13) between said internal and external branches (11, 12) which are further joined by a second cross branch (14) forming, with the first cross branch (13) and with a respective initial tract of the internal and external branches (11, 12) comprised between both cross branches (13, 14), a quadrilateral, the position thereof substantially corresponds to the plantar arch portion of the plantar orthosis (1).

10. The plantar orthosis (1) according to any one of the preceding claims, wherein said continuous belt-like strip has a predetermined thickness comprised between 1.0 mm and 2.2 mm.

11. The plantar orthosis (1) according to any one of the preceding claims, wherein said reinforcement layer (5) is made of thermoplastic-resin-based material, and has a thickness comprised between 0.8 mm and 1.8 mm.

12. The plantar orthosis (1) according to any one of the preceding claims, comprising supporting elements (6), positioned in the heel portion and in the metatarsus portion, implemented by bearings made of low-density soft gum-like material, positioned between the intermediate layer (2) and the interlocking covering layer (3) in suitable recesses (7) or shaped cuts on the base layer (2), the thicknesses thereof correspond to or overcome that of the base layer (2).

13. The plantar orthosis (1) according to any one of the preceding claims, comprising a variable number of pressure (20) and/or deformation sensors.

14. The plantar orthosis (1) according to any one of the preceding claims, comprising a three-axis accelerometer, preferably positioned at the plantar arch portion.

15. Footwear, comprising a plantar orthosis according to any one of the preceding claims, integrated in its own sole.

## Patentansprüche

1. Sohlenorthese (1) zum Stützen des Sohlenbogens eines Benutzers,
wobei ein Fersenabschnitt und ein Mittelfußabschnitt identifiziert werden, die durch einen Zwischenabschnitt des Sohlenbogens getrennt sind, aufweisend:
• eine Deckschicht (2), die zur Aufnahme des ruhenden Fußes des Benutzers bestimmt ist;
• eine untere Abschlussschicht (4);
• eine Basisverstärkungsschicht (5), die zwischen der Deckschicht (2) und der unteren Schicht (4) liegt und die dynamischen Bewegungen der Kompression, der Streckung und der Verdrehung des Sohlenbogens bei der nichtlinearen Aktion der schraubenförmige Auf-/Abwicklung der Pronosupination davon unterstützt und führt;
• ein dynamisches Element (8), das zwischen der Basisverstärkungsschicht (5) und der unteren Schicht (4) positioniert ist, das durch einen kontinuierlichen gürtelartigen Streifen gebildet wird, der in der Nähe der Außenkante des Fersenabschnitts angeordnet ist und der sich mit einem oder mehreren gürtelartigen Zweigen bis zu den jeweiligen vorderen Auflageabschnitten von der Sohlenorthese (1) am äußeren Mittelfuß und in der Nähe des Hallux des Sohlenbogens erstreckt,
wobei das kontinuierliche dynamische Element (8) aus elastischem Material hergestellt ist, das in einem größeren Ausmaß verformbar ist als sowohl die Basisverstärkungsschicht (5) als auch die untere Schicht (4), die es enthält, und wobei der kontinuierliche gürtelartige Streifen elastisch verformt und seine eigenen Abmessungen in den verschiedenen Richtungen verformt, als Ergebnis der nichtlinearen Aktion der schraubenförmigen Auf-/Abwicklung der Pronosupination, indem darin eine druckelastische Welle entlang seiner Länge erzeugt und später in seine eigene natürliche Form zurückkehrt, um elastische mechanische Energie vom Fersenabschnitt zum Mittelfußabschnitt und umgekehrt gemäß privilegierter Richtungen zu speichern, zu übertragen, zu adressieren und freizusetzen.

2. Sohlenorthese (1) nach Anspruch 1, wobei die Basisverstärkungsschicht (5) mit einem strukturellen Bogenverstärkungselement (15) gekoppelt ist, die Dicke der Basisverstärkungsschicht (5) nur auf den Sohlenbogenabschnitt der Sohlenorthese (1) erhöht wird und der Fersenabschnitt und der Bereich, auf dem der Mittelfuß ruht, verbunden wird.

3. Sohlenorthese (1) nach Anspruch 2, wobei das
Bogenverstärkungselement (15) zwischen der Basisverstärkungsschicht (5) und der Deckschicht (2) angeordnet ist.

4. Sohlenorthese (1) nach Anspruch 3, **dadurch gekennzeichnet, dass** das Bogenverstärkungselement (15) mit der Basisverstärkungsschicht (5) und dem dynamischen Element (8) gekoppelt ist, um zusammen mit diesen eine differenzierte Verformung der gesamten Sohlenorthese gemäß Verformungslinien zu bestimmen, die mit den korrekten podalischen Bewegungen kohärent sind, die durch die komplexe Bewegung definiert sind, die durch die rollende Zusammensetzung des ruhenden Fußes und der podalischen Pronations-/Supinationshelix definiert sind.

5. Sohlenorthese (1) nach Anspruch 1, wobei der gürtelartige Streifen am Fersenbereich der Sohlenorthese (1) einen Ring (9) bildet, der nahe den Kanten des Fersenbereichs angeordnet ist.

6. Sohlenorthese (1) nach Anspruch 5, wobei das dynamische Element (8) verbindet, indem es am Sohlenbogenabschnitt eine X-förmige Kreuzung (10) bildet, von der sich ein erster innerer Zweig (11) in Richtung des fünften Mittelfußes und ein zweiter äußerer Zweig (12) in Richtung des ersten Mittelfußes des Benutzers erstreckt, wobei sich ein Ende im Wesentlichen entlang des gesamten ersten Mittelfußes erstreckt.

7. Sohlenorthese (1) nach Anspruch 6, wobei sich der innere Zweig (11) erstreckt bis zur Überwindung der Grenze zwischen Ferse und Mittelfuß des Fußes des Benutzers, und ein Endstück nahe einer solchen Grenze hat.

8. Sohlenorthese (1) nach Anspruch 6, wobei sich der innere Zweig (11) auf den gesamten Mittelfußknochen des fünften Mittelfußes des Fußes des Benutzers erstreckt.

9. Sohlenorthese (1) nach Anspruch 6, wobei der Ring (9) geschlossen ist wiederum durch einen ersten Kreuzzweig (13) zwischen dem inneren und dem äußeren Zweig (11, 12), die außerdem durch einen zweiten Kreuzzweig (14) verbunden sind, der mit dem ersten Querzweig (13) und mit einem jeweiligen Anfangsteil des inneren und des äußeren Zweigs (11, 12), der zwischen beiden Kreuzzweigen (13, 14) liegt, ein Viereck bildet, dessen Position im Wesentlichen dem Sohlenbogenabschnitt der Sohlenorthese (1) entspricht.

10. Sohlenorthese (1) nach einem der vorhergehenden Ansprüche,
wobei der kontinuierliche gürtelartige Streifen eine vorbestimmte Dicke zwischen 1.0 mm und 2.2 mm aufweist.

11. Sohlenorthese (1) nach einem der vorhergehenden Ansprüche,
wobei die Verstärkungsschicht (5) aus einem Material auf Thermoplastharzbasis hergestellt ist und eine Dicke zwischen 0.8 mm und 1.8 mm aufweist.

12. Sohlenorthese (1) nach einem der vorhergehenden Ansprüche, aufweisend Stützelemente (6), die positioniert sind im Fersenbereich und im Mittelfußbereich, implementiert durch Lager aus weichem, gummiartigem Material geringer Dichte, positioniert zwischen der Zwischenschicht (2) und der ineinandergreifenden Deckschicht (3) in geeigneten Aussparungen (7) oder Formschnitten in der Basisschicht (2), deren Dicken denen der Basisschicht (2) entsprechen oder diese übertreffen.

13. Sohlenorthese (1) nach einem der vorhergehenden Ansprüche, aufweisend einer variablen Anzahl von Druck- (20) und/oder Verformungssensoren.

14. Sohlenorthese (1) **nach** einem der vorhergehenden Ansprüche, aufweisend einem dreiachsigen Beschleunigungsmesser, der vorzugsweise im Sohlenbogenabschnitt angeordnet ist.

15. Schuhwerk, das eine in die eigene Sohle integrierte Sohlenorthese nach einem der vorhergehenden Ansprüche aufweist.

## Revendications

1. Orthèse plantaire (1), pour soutenir la voûte plantaire d'un utilisateur, dans laquelle une portion talon et une portion métatarse sont identifiées, séparées par une portion intermédiaire de la voûte plantaire, comprenant :
- une couche de couverture (2) prévue pour recevoir le pied d'appui d'un utilisateur ;
- une couche inférieure de finition (4) ;
- une couche de renforcement de base (5), intermédiaire entre ladite couche de couverture (2) et la couche inférieure (4), supportant et guidant les mouvements dynamiques de compression, d'extension et torsion de la voûte plantaire dans l'action non linéaire de celle-ci d'enroulement/déroulement hélicoïdal de pronosupination ;
- un élément dynamique (8), positionné entre la couche de renforcement de base (5) et la couche inférieure (4), qui est formé par une bande de type courroie continue, agencée à proximité du bord externe de la portion talon, et qui s'étend avec une ou plusieurs branches de type courroie aussi loin que des portions d'appui avant respectives de l'orthèse plantaire (1) au niveau de la partie centrale externe et à proximité de l'hallux de ladite voûte plantaire,
dans laquelle l'élément dynamique continu (8) est constitué de matériau élastique qui est déformable dans une plus grande mesure que la couche de renforcement de base (5) et la couche inférieure (4) le comportant, et dans laquelle ladite bande de type courroie continue se déforme élastiquement et fait varier ses propres dimensions dans les différentes directions, du fait de ladite action non linéaire d'enroulement/déroulement hélicoïdal de pronosupination, en générant à l'intérieur de celui-ci une onde de pression élastique le long de sa longueur, puis en revenant à sa propre forme naturelle, de façon à stocker, transférer, traiter et libérer l'énergie mécanique élastique de la portion talon à la portion métatarse et vice-versa selon des directrices privilégiées préférentielles.

2. Orthèse plantaire (1) selon la revendication 1, dans laquelle ladite couche de renforcement de base (5) est couplée à un élément de renforcement de voûte structurel (15), augmentant l'épaisseur de la couche de renforcement de base (5) de façon limitée à la portion de voûte plantaire de l'orthèse plantaire (1), joignant la portion talon et la portion sur laquelle repose le métatarse.

3. Orthèse plantaire (1) selon la revendication 2, dans laquelle ledit élément de renforcement de voûte (15) est positionné entre la couche de renforcement de base (5) et la couche de couverture (2).

4. Orthèse plantaire (1) selon la revendication 3, dans laquelle l'élément de renforcement de voûte (15) est couplé à la couche de renforcement de base (5) et à l'élément dynamique (8) de façon à déterminer, le long de ceux-ci, une déformation différenciée de toute l'orthèse plantaire selon des lignes de déformation cohérentes avec les mouvements podaliques corrects définis par un mouvement complexe défini par la composition de roulement du pied d'appui et de l'hélice podalique de pronation/supination.

5. Orthèse plantaire (1) selon la revendication 1, dans laquelle ladite bande de type courroie, au niveau de la portion talon de l'orthèse plantaire (1), forme un anneau (9) agencé à proximité des bords de ladite portion talon.

6. Orthèse plantaire (1) selon la revendication 5, dans laquelle l'élément dynamique (8) se joint en formant, au niveau de la portion de voûte plantaire, une traverse en X (10) à partir de laquelle une première branche interne (11) s'étend vers le cinquième métatarse, et, une seconde branche externe (12) s'étend vers le premier métatarse du pied de l'utilisateur, avec une extrémité s'étendant sensiblement le long de l'intégralité du premier métatarse.

7. Orthèse plantaire (1) selon la revendication 6, dans laquelle la branche interne (11) s'étend jusqu'à dépasser la frontière entre le talon et le métatarse du pied de l'utilisateur, et présente une extrémité terminale à proximité de cette frontière.

8. Orthèse plantaire (1) selon la revendication 6, dans laquelle la branche interne (11) s'étend pour tout l'os métatarsien du cinquième métatarse du pied de l'utilisateur.

9. Orthèse plantaire (1) selon la revendication 6, dans laquelle l'anneau (9) est fermé à nouveau par une première branche transversale (13) entre lesdites branches interne et externe (11, 12) qui sont en outre jointes par une seconde branche transversale (14) formant, avec la première branche transversale (13) et avec un tronçon initial respectif des branches interne et externe (11, 12) compris entre les deux branches transversales (13, 14), un quadrilatère, dont la position correspond sensiblement à la portion de voûte plantaire de l'orthèse plantaire (1).

10. Orthèse plantaire (1) selon l'une quelconque des revendications précédentes, dans laquelle ladite bande de type courroie continue présente une épaisseur prédéterminée comprise entre 1,0 mm et 2,2 mm.

11. Orthèse plantaire (1) selon l'une quelconque des revendications précédentes, dans laquelle ladite couche de renforcement (5) est constituée de matériau à base de résine thermoplastique et présente une épaisseur comprise entre 0,8 mm et 1,8 mm.

12. Orthèse plantaire (1) selon l'une quelconque des revendications précédentes, comprenant des éléments de support (6), positionnés dans la portion talon et dans la portion métatarse, mis en oeuvre par des appuis constitués de matériau de type gomme souple à faible densité, positionnés entre la couche intermédiaire (2) et la couche de couverture de verrouillage (3) dans des évidements (7) adaptés ou des découpes formées sur la couche de base (2), l'épaisseur de ceux-ci correspond à ou dépasse celle de la couche de base (2).

13. Orthèse plantaire (1) selon l'une quelconque des revendications précédentes, comprenant un nombre variable de capteurs de pression (20) et/ou de déformation.

14. Orthèse plantaire (1) selon l'une quelconque des revendications précédentes, comprenant un accéléromètre trois axes, de préférence positionné au niveau de la portion de voûte plantaire.

15. Article chaussant, comprenant une orthèse plantaire selon l'une quelconque des revendications précédentes, intégrée dans sa propre semelle.
